(19) Europäisches Patentamt
European Patent Office
Office européen des brevets

(11) Numéro de publication: **0 232 693**
**A2**

(12) # DEMANDE DE BREVET EUROPEEN

(21) Numéro de dépôt: **86870179.8**

(51) Int. Cl.⁴: **A61K 31/475** , A61K 47/00

(22) Date de dépôt: **03.12.86**

Le titre de l'invention a été modifié (Directives relatives à l'examen pratiqué à l'OEB, A-III, 7.3)

(30) Priorité: **16.12.85 LU 86212**
**16.07.86 LU 86515**

(43) Date de publication de la demande:
**19.08.87 Bulletin 87/34**

(84) Etats contractants désignés:
**AT BE CH DE ES FR GB GR IT LI LU NL SE**

(71) Demandeur: **OMNICHEM Société anonyme**
**12 Avenue de Broqueville**
**B-1050 Bruxelles(BE)**

(72) Inventeur: **Hannart, Jean Alfred**
**25, Avenue D'El Pirere**
**B-1302 Dion-Valmont(BE)**
Inventeur: **Trouet, André Benoit Léon**
**29, Predikherenberg**
**B-3009 Winksele(BE)**
Inventeur: **Rao, Kandukuri Sivaprasada Bushana**
**11, rue Kwakembienne**
**B-1331 Rosieres(BE)**

(74) Mandataire: **Van Malderen, Michel et al**
**p.a. Freylinger & Associés 22 avenue J.S. Bach (bte 43)**
**B-1080 Bruxelles(BE)**

(54) **Conjugués de la vinblastine et de ses dérivés, procédé pour leur préparation et compositions pharmaceutiques les contenant.**

(57) On décrit des conjugués d'alcaloïde vinca de type indole-dihydroindole avec une protéine ou un fragment de protéine, répondant à la formule générale

EP 0 232 693 A2

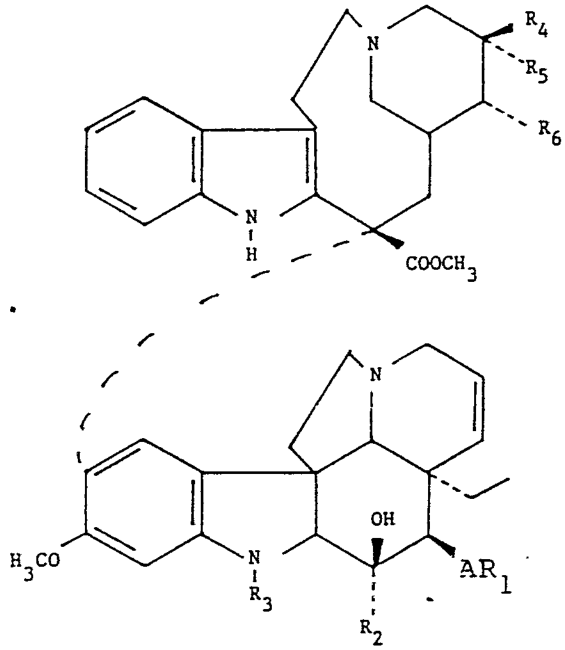

dans laquelle $R_1$ représente une protéine ou un fragment de protéine;
$R_2$ est $COOC_{1-3}$ alkyle, ou $CO-R_7$ où $R_7$ est $NH_2$, un ester d'acide aminé ou de peptide; $R_3$ est H, $CH_3$, CHO;
lorsque $R_5$ et $R_6$ sont pris séparément, $R_6$ est H et un des $R_4$ et $R_5$ est éthyl et l'autre est H ou OH;
lorsque $R_5$ et $R_6$ sont pris ensemble avec les carbones auxquels ils sont attachés, ils forment un noyau oxirane et $R_4$ est éthyle et
A est un dérivé organique bifonctionnel du type maléoylaminoacide ou maléoylpeptide ou maléoylphénoxy.

**NOUVEAUX CONJUGUES DE LA VINBLASTINE ET DE SES DERIVES, PROCEDE POUR LEUR PREPARATION ET COMPOSITIONS PHARMACEUTIQUES LES CONTENANT.**

La présente invention se rapporte à de nouveaux conjugués d'alcaloïdes vinca de type indole-dihydroindole avec des protéines ou des fragments de protéines doués de propriétés pharmaceutiques et, en particulier, d'activité cytostatique. Les alcaloïdes vinca de type indole-dihydroindole, notamment la vinblastine, la vincristine et la vindésine, sont employés dans le traitement du cancer. L'usage chimiothérapeutique de ces dérivés est toutefois limité dans son efficacité par leurs effets secondaires. C'est pourquoi de nombreux dérivés ont été synthétisés afin de diminuer ces effets secondaires.

La vinblastine et certains de ses dérivés, en particulier la vincristine ou la vindésine, ont déjà été couplés à des protéines par exemple l'albumine ou diverses immunoglobulines. Il en résulte des produits de couplage ou des composés dits "conjugués".

Nous relevons notamment les références suivantes de la littérature :

-J.D.Teale, Jacqueline M.Clough et V.Marks, Br.J.Clin.Pharmac. 4, 169-172 ,1977

-C.H.J.Ford, C.E.Newman, J.R.Johnson, C.S.Woodhouse, T.A.Reeder, G.F.Rowland, R.G. Simmonds, Br.J.Cancer 47, 35-42, 1983

-M.J.Embleton, G.F.Rowland, R.G.Simmonds, E.Jacobs, C.H.Marsden, R.W.Baldwin, Br.J.Cancer 47,43-49, 1983

-J.R.Johnson, C.H.J.Ford, C.E.Newman, C.S.Woodhouse, G.F. Rowland, R.G.Simmonds, Br.J.Cancer , 44, 472-475, 1981

-Eli Lilly Eur. Pat. Applic., Publ. n°56.322, 21.07.82

-R.A. Conrad, G.J.Cullinan, K.Gerzon, G.A.Poore, J.Med.Chem. 22, 391, 1979

-Eli Lilly, U.K. Pat. Applic., Publ. n°2.137210, 03.11.84

-OmniChem, Eur. Pat. Applic., Publ. n°124.502, 07.11.84.

Le couplage de ces dimères indole-dihydroindoles a été entrepris non seulement dans le but de mettre au point de nouveaux réactifs immunologiques mais surtout en vue de la préparation de substances antitumorales plus actives, plus sélectives et moins toxiques.

Deux types de couplage ont été jusqu'à présent envisagés.

a) couplage en $C_3$ par l'intermédiaire d'un dérivé azide (Eur. Pat. Appl., Publ. n°56.322).

b) couplage en $C_4$ par l'intermédiaire d'un groupe ester dérivé du groupe hydroxyle du carbone 4 du squelette de l'alcaloïde vinca (U.K. Pat. Appl., Publ. n°2.137.210; Eur. Pat. Appl. n°124.502).

La présente invention concerne des produits nouveaux qui sont des conjugués d'alcaloïdes vinca du type indole-dihydroindole avec des protéines, des fragments de protéines caractérisés en ce que le couplage est effectué également par l'intermédiaire d'un groupe ester dérivé du groupe hydroxyle du carbone 4 du squelette de l'alcaloïde vinca.

L'objet de l'invention consiste plus particulièrement en des conjugués d'alcaloïde vinca de type indole-dihydroindole avec une protéine ou un fragment de protéine, répondant à la formule générale

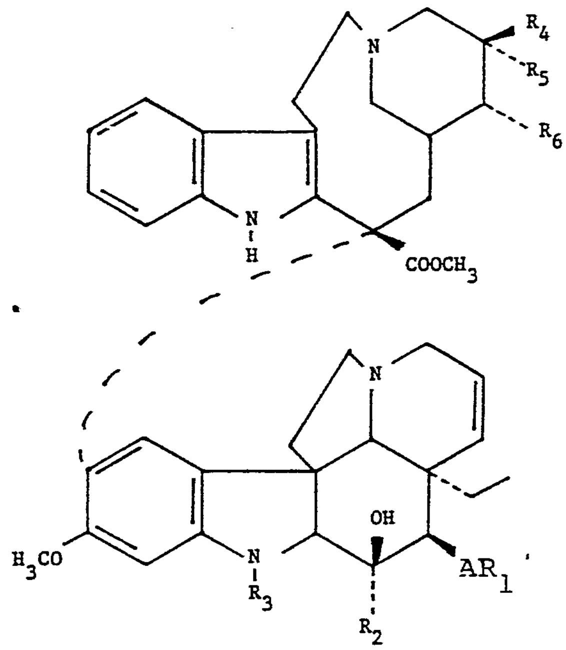

dans laquelle $R_1$ représente une protéine ou un fragment de protéine;
$R_2$ est $COOC_{1-3}$ alkyle, ou CO-$R_7$ où $R_7$ est $NH_2$, un ester d'acide aminé ou de peptide;
$R_3$ est H, $CH_3$, CHO;
lorsque $R_5$ et $R_6$ sont pris séparément, $R_6$ est H et un des $R_4$ et $R_5$ est éthyl et l'autre est H ou OH;
lorsque $R_5$ et $R_6$ sont pris ensemble avec les carbones auxquels ils sont attachés, ils forment un noyau oxirane et $R_4$ est éthyle et
A est un reste de dérivé organique bifonctionnel du type maléoylaminoacide ou maléoylpeptide ou maléoylphénoxy.

Ces conjugués, suivant l'invention, sont caractérisés en ce que la protéine ou le fragment de protéine est couplé au composé vinca par l'intermédiaire d'un bras, par condensation préalable de l'alcaloïde vinca 4-désacétyl indole dihydroindole avec un dérivé organique bifonctionnel de type maléoylaminoacide ou de type maléoylpeptide de formule générale

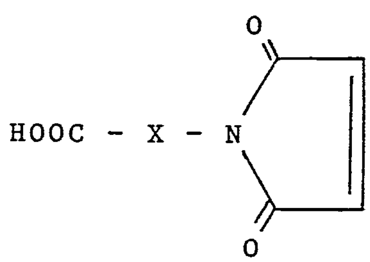

-Dans le cas des maléoylaminoacides
X représente une chaîne linéaire alkylène de 1 à 12 atomes de carbone.
une chaîne ramifiée alkylène de 2 à 5 atomes de carbone.
une chaîne cycloalkylène de 3 à 6 atomes de carbone.
une chaîne phénylène de 3 à 6 atomes de carbone.

De plus, lorsque le dérivé bifonctionnel est de type maléoylaminoacide, X représente, outre les valeurs reprises ci-dessus, le groupement R-CH des acides aminés naturels

R-CH-COOH.
$NH_2$

. Dans ce derniers cas, il va de soi que, lorsque le groupement R-CH est fonctionnalisé, les fonctions peuvent être protégées par les groupements protecteurs habituellements utilisés en synthèse peptidique.

-Dans le cas des maléoylpeptides

X représente un fragment de chaîne peptidique $-(X_1-NH-CO)_n-X_1-$où n = 1,2,3,4 et $X_1$ a la même signification que le X décrit c-dessus.

-X peut également représenter un radical phényl :

Lorsque le dérivé bifonctionnel défini ci-dessus représente des centres d'asymétrie, il peut être utilisé sous sa forme racémique ou sous une de ses formes optiquement actives.

Plus particulièrement, les conjugués de l'invention peuvent être représentés par la formule suivante :

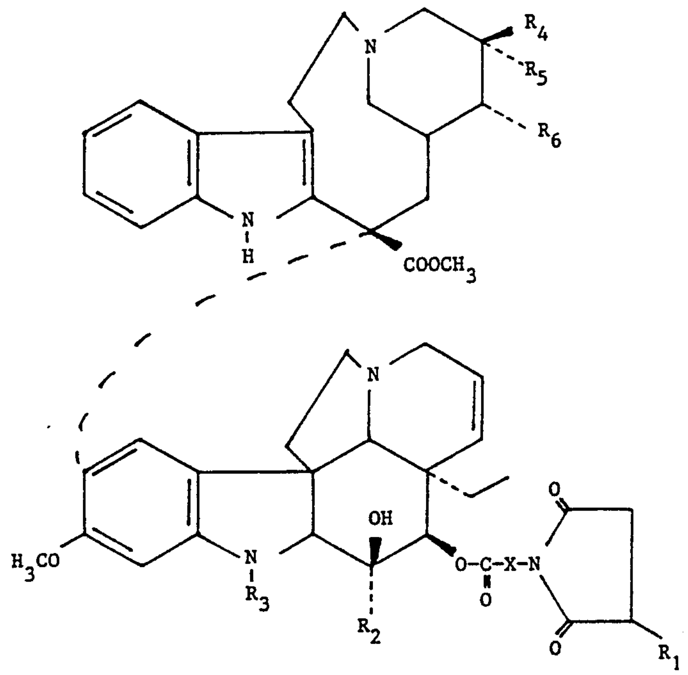

I

dans laquelle $R_1$ représente une protéine ou un fragment de protéine.

X est tel que défini ci-dessus

$R_2$ est $COOC_{1-3}$ alkyl, ou $CO-R_7$ où $R_7$ est $NH_2$ un ester d'acide aminé ou de peptide.

$R_3$ est H, $CH_3$, CHO.

lorsque $R_5$ et $R_6$ sont pris séparément, $R_6$ est H et un des $R_4$ et $R_5$ est ethyle et l'autre est H ou OH.

lorsque $R_5$ et $R_6$ sont pris ensemble avec les carbones auxquels ils sont attachés, ils forment un noyau oxirane et $R_4$ est éthyle.

Les composés ayant la formule précédente peuvent être décrits de manière générique soit comme des dérivés de la vinblastine où $R_2$ est $COOCH_3$, $R_3$ est méthyle, $R_4$ est hydroxyle, $R_5$ est éthyle, $R_6$ est hydrogène.

des dérivés de la vindésine où $R_2$ est $CO-NH_2$, $R_3$, $R_4$, $R_5$ et $R_6$ ont la signification décrite pour les dérivés de la vinblastine.

des dérivés de vinblastinoyl-23 amino acide où $R_2$ est $CO-R_7$, $R_7$ étant un ester d'acide aminé ou de peptide, $R_3$, $R_4$, $R_5$ et $R_6$ ont la signification décrite pour les dérivés de la vinblastine.

des dérivés de la vincristine où $R_2$ est $COOCH_3$, $R_3$ est formyle, $R_4$ est hydroxyle, $R_5$ est éthyle, $R_6$ est hydrogène.

des dérivés de la leurosidine où $R_2$ est $COOCH_3$, $R_3$ est méthyle, $R_4$ est éthyle, $R_5$ est hydroxyle, $R_6$ est hydrogène.

des dérivés de 4-déoxy VLB "A" où $R_2$ est $COOCH_3$, $R_3$ est méthyle, $R_4$ et $R_6$ sont des hydrogènes, $R_5$ est éthyle.

des dérivés de 4-déoxy VLB "B" où $R_2$ est $COOCH_3$, $R_3$ est méthyle, $R_4$ est éthyle, $R_5$ est $R_6$ sont des hydrogènes.

des dérivés de la leurosine où $R_2$ est $COOCH_3$, $R_3$ est méthyle, $R_4$ est éthyle, $R_5$ et $R_6$ forment ensemble un lien -époxide.

Le dérivé bifonctionnel de type maléoylaminoacide peut résulter de la condensation d'une N-alkyoxymaléimide avec un acide aminé naturel ou non, dans le cas de la condensation avec la glycine X = $CH_2$; avec l'alaline X = $CH-CH_3$; avec la $\beta$-alanine X = $(CH_2)_2$; avec la phénylalanine X = $CH-CH_2-C_6H_5$; avec l'acide $\alpha$ -amino butyrique X = $CH-CH_2-CH_3$; avec la valine X = $CH-CH-(CH_3)_2$; avec la norvaline X = $CH-CH_2-CH_2-CH_3$; avec la leucine X = $-CH-CH_2-CH-(CH_3)_2$; avec

l'isoleucine X = 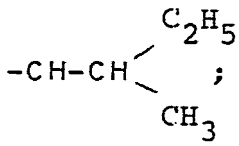 ; avec la norleucine X =

$CH-(CH_2)_3-CH_3$; avec l'acide 6-amino caproïque X = $(CH_2)_5$; avec l'acide 11-aminoundecanoïque X = $(CH_2)_{10}$; avec l'acide 12-aminododecanoïque X = $(CH_2)_{11}$.

Le dérivé bifonctionnel de type maléoylpeptide peut résulter également de la condensation d'une N-alkoxy maléimide avec un dipeptide, un tripeptide pour donner un dérivé $-(X_1-NH-CO-)_nX_1$ où $X_1$ peut avoir la même signification que le X décrit ci-dessus.

Les protéines qui peuvent être avantageusement utilisées sont en particulier l'albumine de sérum bovin ou humain, la fétuine ou des immunoglobulines.

Les protéines utilisées peuvent également être traitées afin d'être sélectivement modifiées. Ces modifications permettent d'obtenir des conjugués protéiques qui seront, lors de leur utilisation thérapeutique, préférentiellement concentrés au niveau de certains tissus, par exemple, au niveau du foie. Il est ainsi possible, préalablement à la condensation du dérivé de l'alcaloïde vinca à la protéine, de galactosyler cette dernière.

Les immunoglobulines spécifiques aux antigènes de surface de cellules malignes et les techniques pour leur production à partir de sérum d'animaux immunisés ou par culture d'hybridomes secrétant des anticorps monoclonaux sont bien connues. Le type préféré d'anticorps pour l'usage dans l'invention est une immunoglobuline de la classe IgG d'origine humaine.

Toutefois, les immunoglobulines d'autres espèces sont aussi inclues dans cette invention. Certaines immunoglobulines représentatives sont les suivantes :

-Ig de chèvre ou de mouton immunisés avec l'antigène carcinoembryonnaire.

-Ig de lapin anti-LLA

-Différentes Ig de singe anti-LLA, anti-LMA, anti-LLC, anti-LMC.

-Ig de chèvre ou de mouton immunisés avec des membranes de carcinome du poumon.

-Ig monoclonales à partir d'hybridomes de souris secrétant des anticorps contre le carcinome colo-rectal humain.

- Ig monoclonales à partir d'hybridomes de souris secrétant des anticorps contre le mélanome humain.

-Ig monoclonales à partir d'hybridomes de souris secrétant des anticorps réagissant avec des cellules leucémiques humaines.

-Ig monoclonales à partir d'hybridomes de souris secrétant des anticorps réagissant avec des cellules de neuroblastomes humaines.

-Ig monoclonales à partir d'hybridomes de souris secrétant des anticorps réagissant avec des antigènes de cancer du sein humain.

-Ig monoclonales à partir d'hybridomes de souris secrétant des anticorps réagissant avec des cellules de carcinome ovarien humain.

-Ig monoclonales à partir d'hybridomes de souris secrétant des anticorps réagissant avec des cellules d'osteosarcome humain.

-Ig monoclonales à partir d'hybridomes de souris secrétant des anticorps du cancer du poumon.

Les conjugués peuvent également être préparés avec des fragments d'immunoglobulines à savoir les fragments Fab, Fab' ou $F(ab')_2$ ou IgM monomère obtenus à partir d'un anticorps par digestion par un enzyme protéolytique.

Les conjugués de la présente invention sont obtenus, dans une première étape, par condensation d'un maléoylaminoacide ou d'un maléoylpeptide sur l'hydroxyle en $C_4$ d'un alcaloïde vinca 4-désacétyl indole-dihydroindole de formule II pour donner un dérivé de formule III,

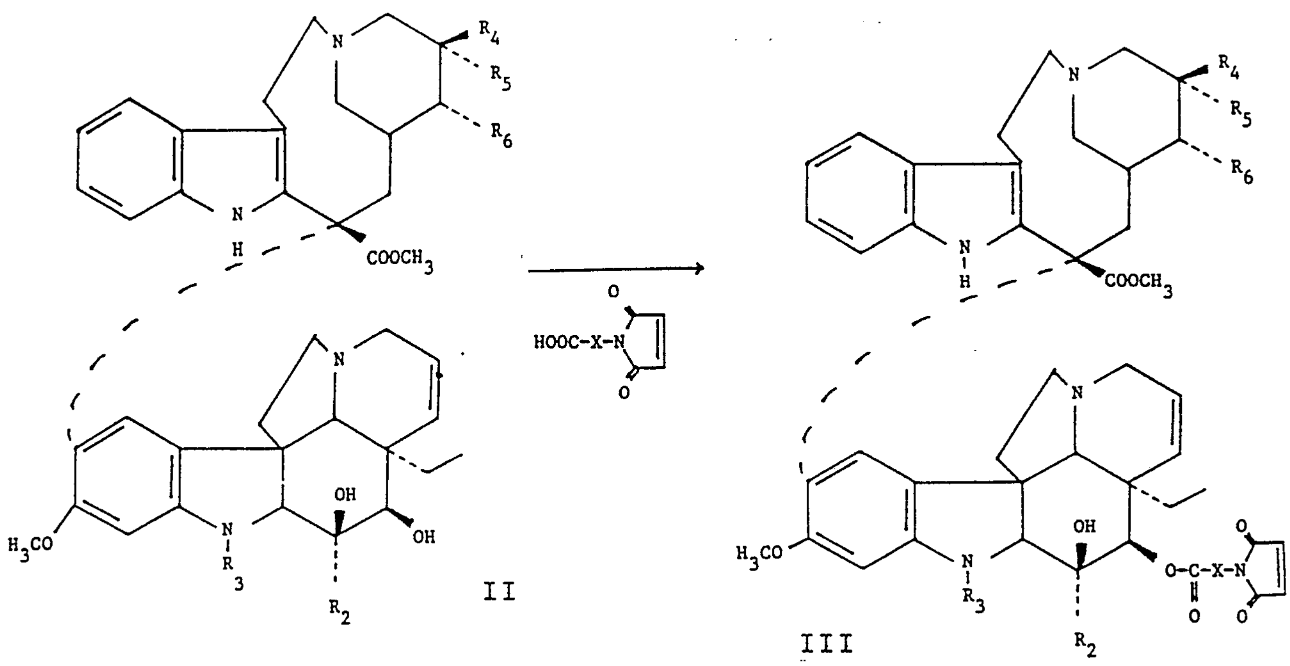

II

III

dans lequel X, $R_2$, $R_3$, $R_4$, $R_5$, $R_6$ ont la signification mentionnée ci-dessus.

Dans une deuxième étape, les dérivés vinca-4-carboxy maléoyl III sont alors condensés avec une protéine ou un fragment de protéines par addition soit des groupement thiols libres, soit des groupements aminés libres de la protéine, par exemple, les groupements aminés dérivés des résidus lysine de la protéine sur la double liaison oléfinique de la maléimide suivant un mécanisme d'addition de type Michael - (Means, G.E. et Feeney, R.E.; Chemical modification of proteins, 1971, p.110-138, Holden Day Inc., San Francisco) pour donner les conjugués I.

Du point de vue chimique, -l'obtention des maléoylaminoacides ou des maléoylpeptides est réalisée selon les méthodes décrites par O. Keller et J. Rudinger, Helv. 58, 531 (1975), par D.H. Rich, P.D. Gesselchen, A. Tong, A. Cheung et C.K. Buckner; J. Med. Chem., 18, 1004 (1975) et par A. Sato et M. Nakao; J. Biochem., 90, 1117 (1981). -la condensation des dérivés maléoyls avec l'alcaloïde vinca peut être effectuée de manière classique par un chloroformiate d'alkyle, de préférence le chloroformiate d'éthyle ou d'isobutyle, en présence d'une base aminée telle que la triéthylamine, la N-méthyl pipéridine, la N-méthyl morpholine, dans un solvant organique tel que l'acétate d'éthyle, le tetrahydrofuranne, le chlorure de méthylène.

Le dérivé obtenu est isolé du milieu réactionnel et purifié au moyen de méthodes classiques utilisées en chimie.

Toute autre méthode d'activation du groupement carboxylique du maléoylamino acide ou du maléoylpeptide pour réaliser la condensation avec l'alcaloïde vinca, en particulier les méthodes utilisées dans la chimie des peptides, peut être appliquée à ce type de condensation.

En particulier, dans le cas où X est phényl, la condensation est réalisée avec le N-succinimidyl-3 maléimidobenzoate, obtenu selon les méthodes décrites par T. Aikawa; J. Biochem., 79, 233 (1976) et M.J. O'Sullivan et al., Anal. Biochem., 100, 100 (1979).

L'obtention des conjugués peut être réalisée en faisant réagir la protéine, l'anticorps polyclonal ou monoclonal avec le composé vinca de formule III sous des conditions classiques, par exemple en milieu aqueux, à une température comprise entre 4°C et 40°C et un pH de 7,5 à 9,5.

Le nombre de résidus attachés peut dépendre de la concentration des réactifs et de la durée de la réaction, mais le nombre moyen est généralement compris entre 5 et 20.

Par exemple, une solution du composé III dans un solvant organique tel que le dioxanne est ajoutée goutte à goutte à une solution tamponnée de protéine, par exemple un tampon phosphate 0,1 M à pH 8,2. Après une nuit à la température ordinaire, le conjugué est isolé par filtration sur gel, concentré par ultrafiltration et stérilisé. Le contenu en protéines est mesuré par la méthode de Lowry et le contenu en alcaloïdes estimé par mesure de la radioactivité.

Les essais "in vitro" et "in vivo" effectués avec les composés de l'invention pour démontrer leur activité anti-tumorale indiquent que la formation du conjugué par l'intermédiaire d'un lien maléoyl

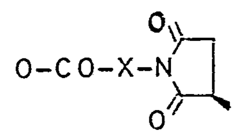

peut être plus avantageuse que par un lien O-CO-X-CO-

En effet, le lien maléoyl permet à la fois, la conjugaison des groupements thiols et des groupements aminés libres de la protéine ou du fragment de protéine. De plus, la digestion par les enzymes lysosomiales indiquent une libération beaucoup plus importante de l'alcaloïde vinca dans le cas du lien maléoyl. Les conjugués sont également stables dans le sérum et à pH acide.

Les composés de l'invention ont été testés sur des souris $BDF_1$ auxquelles une leucémie P388 a été implantée par voie intra-péritonéale. Les premiers résultats indiquent que les composés présentent une activité significative sur ce modèle expérimental puisqu'ils induisent une augmentation du temps de survie.

Les nouveaux conjugués de l'invention présentent des propriétés anti-tumorales particulièrement intéressantes et susceptibles d'être utilisées en thérapeutique humaine.

Pour leur application thérapeutique, les composés de l'invention, sont de préférence administrés par voie parentale, dissous dans un solvant pharmaceutiquement acceptable. L'eau physiologique ou d'autres solutions tamponnées, par exemple avec un phosphate sont des solvants appropriés. La substance active est généralement administrée à une posologie pouvant varier de 50 mg à plusieurs grammes. Les composés de l'invention peuvent en outre être utilisés en combinaison avec d'autres agents anti-tumoraux.

Les exemples suivants illustrent de manière non limitative le procédé conduisant aux composés de l'invention.

Exemple 1 : N-carbomethoxy maléimide.

Une solution de 3,8 g (0,04 M) de maléimide et 4 g (0,04 M) de triethylamine dans 150 ml d'acétate d'éthyle est traitée à 0°C par 3 ml (0,04 M) de méthylchoroformiate dissous dans 20 ml d'acétate d'éthyle. Après une heure d'agitation, le précipité est filtré, lavé par l'acétate d'éthyle. Les phases organiques sont rassemblées, lavées par de l'eau, séchées sur sulfate de sodium anhydre et évaporées à sec sous vide. Le résidu, cristallisé dans un mélange acétate d'éthyle-éther isopropylique, donne 4,19 g de N-carbomethoxy maléimide.

Spectre RMN : ($CDCl_3$,$\delta$) : 6,75 (2H,s), 3,9(3H,s)

Exemple 2 : N-Maléoyl L-alanine.

Une solution de 2,3 g (0,025 M) de L-alanine dans 100 ml d'une solution saturée de bicarbonate de sodium est traitée à 0°C, sous agitation vigoureuse, par 3,87 g (0,025 M) de N-carbométhoxymaléimide. Après une heure, la solution est diluée par 200 ml d'eau et agitée à 40°C pendant une heure. Le pH de la solution (8,2) est ensuite amené à 6,4 par addition d'acide sulfurique concentré. La solution est ensuite concentrée à 100 ml et acidifiée à pH 2 par addition d'acide sulfurique 1 M et extraite trois fois par l'acétate d'éthyle. Les phase organiques sont rassemblées, séchées sur sulfate de sodium anhydre et évaporées à sec sous vide.

Le résidu obtenu est purifié par chromatographie sur colonne de silice (élution chloroforme/acide acétique 95/5). On obtient de cette façon 1,2 g de N-maléoyl L-alanine.

Rendement 48%.

Spectre I.R. (KBr) $cm^{-1}$ : 3400, 3100. 2930, 1780, 1740, 1700, 1460, 1420, 1390, 1370, 1345, 1220, 1175, 1118, 1080, 1068, 1015, 970, 835, 700.

Spectre R.M.N. ($CDCl_3$)$\delta$: 6,7 (2H,s); 4,8 (1H,g); 1,65 (3H,d)

Exemple 3 : acide N-maleoyl-6-aminocaproïque

Une solution de 4.2 gr (32.3 mM) d'acide 6-amino caproïque dans 163 ml d'une solution saturée de bicarbonate de sodium est traitée à 0°C, sous agitation vigoureuse, par 5 gr (32,3 mM) de N-carbométhoxymaléimide. Après 1 heure, la solution est diluée par 256 ml d'eau et agitée à température ambiante pendant 1 heure. Le pH de la solution (8,2) est ensuite amené à 6,4 par addition d'acide

sulfurique concentré. La solution est ensuite concentrée à 100 ml et acidifiée à pH 2 par addition d'acide sulfurique 1M et extraite trois fois par l'acétate d'éthyle. Les phases organiques sont rassemblées, séchées sur sulfate de sodium anhydre et évaporées à sec sous vide. Le résidue obtenu est purifié par chromatographie sur colonne de silice (élution chloroforme/acide acétique 95/5). On obtient de cette façon 4.7 gr d'acide N-maléoyl-6-amino caproïque.

Rendement : 51%.

Spectre I.R. (KBr/$cm^{-1}$) : 3.090; 2.940; 2.880; 1.770; 1.710; 1.470; 1.450; 1.380; 1.370; 1.340; 1.315; 1.260; 1.210; 1.135; 1.110; 1.140; 1.100; 1.015; 1.000; 815; 840; 735; 700.

Exemple 4 : Acide (N-maleoyl)-L-glutamique$_\gamma$ méthyl ester

Une solution de 1 gr (6,2 mM) d'acide L-glutamique $_\gamma$ méthyl ester dans 31,2 ml d'une solution saturée de bicarbonate de sodium est traitée à 0°C sous agitation vigoureuse par 961 mg (6,2 mM) de N-carbométhoxymaléimide. Après une heure, la solution est diluée par 126 ml d'eau et agitée à 40°C pendant une heure. Le pH de la solution (8,2) est ensuite amené à 6.4 par addition d'acide sulfurique concentré. La solution est ensuite concentrée à 50 ml et acidifiée à pH 2 par addition d'acide sulfurique 1 M et extraite trois fois par l'acétate d'éthyle. Les phases organiques sont rassemblées, séchées sur sulfate de sodium anhydre et évaporées à sec sous vide. Le résidu obtenu est purifié par chromatographie sur colonne de silice (élution chloroforme/acide acétique 95/5). On obtient de cette façon 956 mg de produit pur.

Rendement : 49%.

Spectre R.M.N 60 : 7,9 (s, H acide) ; 6,5 (s, maléimide) ; 4,6 (m, CH*) ; 3,5 (s, $OCH_3$) ; 2,3 (m, $CH_2$ glu).

Spectre I.R. (KBr) $cm^{-1}$ : 3.470, 3.110, 2.960, 2.600, 1.775, 1.750 -1.690, 1.440, 1.410, 1.265-1.150, 1.090, 1.015, 830, 700.

Exemple 5 : N-maléoyl-L-isoleucine

Suivant le mode opératoire de l'exemple 2, on obtient le N-maléoyl-L-isoleucine en traitant 424 mg - (3,22 mM) de L-isoleucine par 500 mg (3,2 mM) de N-carbométhoxymaléimide.

Rdt : 20%.

Spectre de masse (CDI, isobutane) : 423 (2M +1), 352, 270, 212 ($M^+$ + 1), 188, 166, 132, 123.

Spectre R.M.N. ($CDCl_3$)$\delta$: 8.55 (ép, OH) : 6.85 (2, s, double liaison maléimide) ; 4.65 (1, d, C*H) ; 2.6 (m, 1, CH) ; 1.28 (d, $CH_3$) ; 1.1 (m, $CH_3$).

Exemple 6 : Acide N-Maleoyl-L-aspartique $\alpha$ Benzyl ester.

Une solution de 1 gr (4.48 mM) d'acide N-Maleoyl-L-aspartique $\alpha$ Benzyl ester dans 22.5 ml d'une solution saturée de bicarbonate de sodium est traitée à 0°C sous agitation vigoureuse par 694 mg (4.48 mM) de N-carbomethoxymaléimide. -

Après 1 h, la solution est diluée par 91 ml d'eau et agitée à 40°C pendant une heure. Le pH de la solution (8.2) est ensuite amené à 6.4 par addition d'acide sulfurique concentré. La solution est ensuite concentrée à 50 ml et acidifiée à pH 22 par addition d'acide sulfurique 1M et extraite trois fois par l'acétate d'éthyle.

Les phases organiques sont rassemblées, séchées sur sulfate de sodium anhydre et évaporées à sec sous vide.

Le résidu obtenu est purifié par chromatographe sur colonne de silice (élution chloroforme / acide acétique 95/5). On obtient de cette façon 267 mg de produit pur.

Rendement : 20%

Spectre RMN ($CD_3OD$, 60 MHz, ppm) :

7,1 (5H, m, H benzyl); 6.6 (2H, s, dl maléimide); 5 (2H, s, $CH_2$ benzyl) 3.1 (2H, m, $CH_2$).

Spectre IR (KBr, $cm^{-1}$) :

3060, 1765, 1745, 1720, 1410, 1270, 830, 740, 700.

Exemple 7 : Acide N-maleoyl-11-amino undecanoïque.

Une solution d'anhydride maléique (2.4 gr, 24.8 mM) dans 10.1 ml d'acide acétique est ajoutée à une solution d'acide 11-amino undecanoïque (5 gr, 24.8 mM) dans 30 ml d'acide acétique et le mélange est maintenu sous agitation vigoureuse à température ambiante pendant 3 heures.

Le précipité blanc est filtré, lavé avec de l'acide acétique froid et séché (6.3 gr, 21.1 mM), 85%). 3 gr du précipité (10.00 mM) sont dissous dans du toluène sec (300 ml) et traités avec de la triéthylamine (2 gr, 22.2 mM).

La solution est portée à reflux dans un appareil Dean Stark sous agitation vigoureuse, jusqu'à évaporation du toluène. Le produit est purifié sur une colonne de silice (élution chloroforme / acide acétique 95/5). On obtient de cette façon 1.06 gr d'acide N-maléoyl-11-amino undecanoïque.

Rendement : 37%

Spectre RMN (60 MHz, $CDCl_3$, ppm) :

8.8 (bp, COOH); 6.5 (2H, s, dl maléimide); 3.4 (2H, m, $CH_2$); 2.3 (2H, m, $CH_2$); 1.2 (16H, bp, $8CH_2$).

Spectre IR (KBr, $cm^{-1}$) :

3450, 3100, 2910, 2850, 1770, 1700, 1610, 1590, 1470, 1450, 1420, 1375, 1340, 1310, 1280, 1240, 1180, 1125, 840, 700.

Exemple 8 : Acide N-maleoyl-12-amino dodecanoïque.

Une solution d'anhydride maléïque (2.28 gr, 23.22 mM) dans 9.6 ml d'acide acétique est ajoutée à une solution d'acide 12 amino dodecanoïque (5 gr, 23.22 mM) dans 28 ml d'acide acétique et le mélange est maintenu sous agitation vigoureuse à température ambiante pendant 3 heures.

Le précipité blanc est filtré, lavé avec l'acide acétique froid et séché (6.2 gr, 20.9 mM, 86%). 4.9 gr du précipité (15.9 mM) sont dissous dans du toluène sec (500 ml) et traités avec de la triéthylamine (4.9 ml, 35 mn). La solution est portée à reflux dans un appareil Dean Stark sous agitation vigoureuse, jusqu'à évaporation du toluène. Le produit est purifié sur une colonne de silice (élution chloroforme / acide acétique 95/5). On obtient de cette façon 1.38 gr d'acide N-maleoyl-12-amino dodecanoïque.

Rendement : 29%

Spectre RMN (60 MHz, $CDCl_3$, ppm) :

6.52 (2H, s, dl maléimide); 3.42 (2H, m, $CH_2$); 2.3 (2H, m, $CH_2$); 1.25 (18H, broad peak, $9-CH_2-$)

Spectre IR (KBr, $cm^{-1}$) :

3450, 3080, 2920, 2825, 1770, 1470, 1450, 1440, 1415, 1380, 1340, 1300, 1260, 1250, 1205, 1120, 920, 840, 700.

Exemple 9 : 4-(N-maléoyl)-L-alanyl-vinblastine.

A une solution de 1,52 g (0,009 M) de N-maléoylalanine et de 1,25 ml (0,009 M) de triéthylamine dans 10 ml d'acétate d'éthyle refroidie à 0°C, on ajoute goutte à goutte une solution de 1,17 ml (0,009 M) d'isobutyl-chloroformiate dans 5 ml d'acétate d'éthyle. On agite 1 h 30 à 0°C, puis on ajoute 2,3 g (0,003 M) de 0-4 deacetyl vinblastine en solution dans 20 ml d'acétate d'éthyle en gardant la température à 0°C. On laisse ensuite revenir à température ambiante et agite le mélange 10 heures. On ajoute 50 ml d'acétate d'éthyle et 50 ml d'une solution aqueuse à 10% de carbonate de sodium. On agite, décante et sépare la phase organique. La phase aqueuse est extraite trois fois par de l'acétate d'éthyle. Les phases organiques réunies sont lavées par une solution aqueuse, séchées sur sulfate de magnésium et évaporées à sec sous vide. Le résidu obtenu est purifié par chromatographie sur colonne de silice (élution dichlorométhane/méthanol 92/8). On obtient ainsi 1,86 g de produit pur.

Rendement : 67,6%

Spectre de masse (DCI, isobutane) : 935 (M+14), 922 (M+1), 921 (M), 751, 693, 519, 445, 371, 133.

Spectre RMN ($CDCl_3$,360 MHz,ppm) : 8 (NH,1H); 7,5-7 (4H, H-9', H-10'H-11', H-12'); 6,7 (2H,anhydride); 6,55 (1H, H-14); 6,05 (1H,H-17); 5,85 (1H,H-7); 5,45 (1H,H-4); 5,15 (1H,H-6); 4,8 (1H, CH*); 3,95 (1H,H-17'); 3,85 (3H, -OMe); 3,78 (3H,-OMe); 3,7 (1H,H-2); 3,6 (3H,-Ome); 2,7 (3H,NMe); 1,72 (3H,$CH_3$ alanine); 0,9-0,8 (6H, $CH_3$-21, $CH_3$-21').

Exemple 10 : 4-(N-maléoyl)-6-amino-caproyl vinblastine

A une solution de 604 mg (2.861 mM) d'acide N-maléoyl-6-amino caproïque et de 514 µl (4,65 mM) de M-méthylmorpholine dans 4 ml d'acétate d'éthyle refroidie à 0°C, on ajoute goutte à goutte une solution de 371 µl (2.861 mM) d'isobutylchloroformiate dans 1 ml d'acétate d'éthyle. On agite 3 min à 0°C, puis on ajoute 550 mg (0,715 mM) de 0-4-déacétyl vinblastine en solution dans 1 ml d'acétate d'éthyle en gardant la température à 0°C. On laisse ensuite revenir à température ambiante et on agite le mélange pendant 10 heures. On filtre la solution et la phase acétate d'éthyle est évaporée à sec sous vide. Le résidu obtenu est purifié par chromatographie sur colonne de silice (élution dichlorométhane/méthanol 96/4). On obtient ainsi 263 mg de produit pur.

Rendement : 23 %.

Spectre de masse (DCI, isobutane): 993 ; 979 ; 965 ($M^+$ +4) ; 963 ($M^+$ +2); 961 ($M^+$) ; 946 ; 933 ; 920 ; 906 ; 812; 754 ; 693.

Spectre I.R. (KBr, $cm^{-1}$) : 3.400 ; 3.050 ; 2.940 ; 1.740 ; 1.700 ; 1.615 ; 1.500 ; 1.460 ; 1.440 ; 1.410 ; 1.370 ; 1.220 ; 1.170 ; 1.040.

Spectre RMN ($CDCl_3$, 360 MHz, ppm) : 7,47-7,12 (4H, m, $H^{11'}$, $H^{12'}$, $H^{13'}$, $H^{14'}$) ; 6,65 (2H, s, d.l.maléimide) ; 6,55 (14, s, $H^{14}$) ; 6,05 (1H, s, $H^{17}$) ; 5,82 (1H, m, $H^7$) ; 5,42 (1H, s, $H^4$) ; 5,25 (1H, m, $H^6$) ; 3,92 (1H, m, $H^{17'}$) ; 3,77 (6H, s, $OCH_3$ $C^{23}OOCH_3$) ; 3,70 (1H, s, $H_2$) ; 3,6 (3H, s, $C^{18'}OOCH_3$) ; 2,7 (3H, s, $NCH_3$) ; 2,32 (m, $CH_2$ aminocaproïc) ; 1.62 (m, $CH_2$ aminocaproïc) ; 0,9-0,8 (6H, t, $CH_3$21 + $CH_3$21').

Exemple 11 : 4-(N-maleoyl)-L-glutamyl γ méthyl ester vinblastine

A une solution de 282 mg (1,170 mM) d'acide N-maleoyl-L-glutamique γméthyl ester et de 163 µl - (1,170 mM) de triéthylamine dans 1,4 ml d'acétate d'éthyle refroidie à 0°C, on ajoute goutte à goutte une solution de 152 µl (1,170 mM) d'isobutylchloroformiate dans 1 ml d'acétate d'éthyle. On agite 4 min à 0°C, puis on ajoute 300 mg (0,390 mM) de 0-4-déacétyl vinblastine en solution dans 1 ml d'acétate d'éthyle en gardant la température à 0°C. On laisse ensuite revenir à température ambiante et on agite le mélange 10 heures. On filtre la solution et la phase organique est concentrée sous vide. Le résidu obtenu est purifié par chromatographie sur colonne de silice (élution éthanol/acétate d'éthyle 30/90). On obtient ainsi 222 mg de produit pur.

Rendement : 38%.

Spectre de masse (DCI, isobutane) : 1.036, 1.022, 1.009, 995 ($M^+$ +4), 992 ($M^+$ +1), 937, 885, 811, 751, 694, 635, 541.

Spectre RMN ($CDCl_3$, 360 MHz, ppm) : 9,4 (1H, m, OH) ; 8 (1H, s, NH ind) ; 7,5-7,10 (4H, m, $H^{11'}$, $H^{12'}$, $H^{13'}$, $H^{14'}$) ; 6,7 (2H, s, dl, maléimide) ; 6,58 (1H, s, $H^{14}$) ; 6,05 (1H, s, $H^{17}$) ; 5,80 (1H, m, $H^7$) ; 5,48 (1H, s, $H^4$) ; 5,25 (1H, m, $H^6$) ; 4,73 (1H, m, CH* glu) ; 3,95 (1H, m, H17') ; 3,85 (3H, s, $OCH_3$ar) ; 3,75 (3H, s, $C^{23}OOCH_3$) ; 3,63 (3H, s, $C^{18'}OOCH_3$) ; 3,58 (3H, s, $OCH_3$ glu) ; 2,80 (3H, s, $NCH_3$) 2,38 (m, $CH_2$ glu) ; 0,9-0,8 (6H, t, $CH_3$ 21 + $CH_3$ 21').

Spectre I.R. (KBr) $cm^{-1}$ : 3.430, 1.740, 1.715, 1.615, 1.500, 1.460, 1.430, 1.405, 1.385, 1.250, 1.225, 1.030, 1.005, 825.

Exemple 12 : 4-(N-maléoyl)-L-Isoleucyl vinblastine

A une solution de 150 mg (0,7109 mM) de N-maléoyl-L-isoleucine et de 99 µl (0,7109 mM) de triéthylamine dans 1 ml d'acétale d'éthyle refroidie à 0°C, on ajoute goutte à goutte une solution de 92 µl - (0,7109 mM) d'isobutylchloroformiate dans 1 ml d'acétate d'éthyle. On agite 3 min à 0°C, puis on ajoute 182 mg (0,237 mM) de 0-4-déacétyl vinblastine en solution dans 1 ml d'acétate d'éthyle en gardant la température à 0°C. On laisse ensuite revenir à température ambiante et on agite le mélange pendant 10 heures. On filtre la solution et la phase acétate d'éthyle est évaporée à sec sous vide. Le résidu obtenu est purifié par chromatographie sur colonne de silice (élution éthanol/acétate d'éthyle 30/90). On obtient ainsi 133 mg de produit pur.

Rdt : 40%.

Spectre de masse (DCI, acétone) : 1.039 ; 963 ($M^+$ + 2) ; 904 ; 812 ; 769 ; 728 ; 637 ; 593 ; 549.

Spectre I.R. (KBr, $cm^{-1}$) : 3.480-3.400 ; 2.960 ; 2.920 ; 2.880 ; 1.775 ; 1.740 ; 1.710 ; 1.610 ; 1.500 ; 1.460 ; 1.430 ; 1.380 ; 1.250 ; 1.220 ; 1.040 ; 1.000 ; 830 ; 740.

Spectre R.M.N. ($CDCl_3$, 360 mHz, ppm) : 9.4 (m, OH) ; 7.5-7.1 (4H, m, CH arom. 11'-12'-13'-14') ; 6.65 - (2H, s, d, liaison maléimide) ; 6.6 (1H, s, $C^{14}$-H) ; 6.05 (1H, s, $C^{17}$-H) ; 5.8 (1H, s, $C^7$-H) ; 5.45 (1H, s, $C^4$-H) ; 5.25 (1H, m, $C^6$-H) ; 4.5 (1H, d, C-H) ; 3.95 (1H, m, $H^{17'}$) ; 3.8 (3H, s, $OCH_3$ ar) ; 3.75 (3H, s, $C^{23}OOCH_3$) ; 3.7 (1H, s, $H_2$) ; 3.6 (3H, s, $C^{18'}OOCH_3$) ; 2.65 (3H, s, $NCH_3$) ; 1.1 (d, 3H, $CH_3$ isoleucine) ; 0.9-0.8 (9H, m, $CH_3$ isoleucine + $CH_3$ 21' + CH-21).

Exemple 13 : 4-(N-Maleoyl)-L-aspartyl α benzyl ester vinblastine.

A une solution de 267 mg (0.88 mM) d'acide N-Maleoyl-L-aspartyl α benzyl ester et de 118 µl (0.88 mM) de triethylamine dans 1 ml d'acetate d'éthyle refroidie à 0°C, on ajoute goutte à goutte une solution de 114 µl (0.88 mM) d'isobutylchloroformiate dans 1 ml d'acétate d'éthyle.

On agite 4 minutes à 0°C, puis on ajoute 227 mg (0.29 mM) de 0-4-deacetyl vinblastine en solution dans 1 ml d'acétate d'éthyle en gardant la température à 0°C. On laisse ensuite revenir à température ambiante et on agite le mélange une nuit.

On filtre la solution et la phase organique est concentrée sous vide. Le résidu obtenu est purifié par chromatographie sur colonne de silice (élution éthanol / acétate d'éthyle 30/90). On obtient ainsi 105 mg de produit pur.

Rendement : 34%

Spectre RMN ($CDCl_3$, 360 MHz, ppm) :

8.05 (1H, s, NHind $^{16'}$); 7.5-7.17 (4H, m, $H^{11'}H^{12'}H^{13'}H^{14'}$); 7.37-7.3 (5H, m, H benzyl); 6.75 (2H, s, dl maléimide); 6.60 (1H, s, $H^{14}$); 6.10 (1H, s, $H^{17}$); 5.87 (1H, m, $H^7$); 5.45 (1H, s, $H^4$); 5.30 (1H, m, $H^6$); 5.20 (2H, m, $CH_2$ benzyl); 3.95 (1H, m, H $^{17'}$); 3.82-3.75 (6H, s, $OCH_3$ + $COOCH_3^{23}$); 3.70 (1H, s, $H_2$); 3.62 (3H, s, $C^{18'}OOCH_3$); 2.60 (3H, s, $NCH_3$); 0.9-0.8 (6H, $tCH_3 21$ + $tCH_3 21'$)

Spectre IR (KBr, $cm^{-1}$)

3460, 3030, 2960, 2880, 1770, 1740, 1715, 1610, 1500, 1460, 1430, 1410, 1380, 1260, 1220, 1175, 1110, 1025, 910, 800, 730, 700

Spectre de masse (DCI, isobutane) :

1085, 1071, 1057, 1054, 1039, 1023, 1013, 768, 708, 542, 158.

Exemple 14 : 4-(N-maleoyl)-11-amino undecanoyl vinblastine.

A une solution de 476 mg (1.69 mM) d'acide N-maléoyl 11-amino undecanoïque et de 326 µl (2.8 mM) de N-methylmorpholine dans 3 ml d'acetate d'éthyle refroidie à 0°C, on ajoute goutte à goutte une solution de 218 µl (1.69 mM) d'isobutylchloroformiate dans 1 ml d'acétate d'éthyle. On agite 3 min. à 0°C, puis on ajoute 433 mg (0.56 mM) de 0-4-deacetyl vinblastine en solution dans 1 ml d'acetate d'éthyle en gardant la température à 0°C. On laisse ensuite revenir à température ambiante et on agite le mélange pendant une nuit.

On filtre la solution et la phase acétate d'éthyle est évaporée à sec sous vide. Le résidu obtenu est purifié par chromatographie sur colonne de silice (élution éthanol / acétate d'éthyle 30/90). On obtient ainsi 240 mg de produit pur.

Rendement : 41%

Spectre RMN ($CDCl_3$, 360 MHz ppm) :

8(1H, s, NH ind $^{16'}$); 7.5-7.13 (4H, m, $H^{11'}H^{12'}H^{13'}H^{14'}$); 6.68 (2H, s, dl maléimide); 6.63 (1H, s, $H^{14}$); 6.10 - (1H, s, $H^{17}$); 5.83 (1H, m, $H^7$); 5.48 (1H, s, $H^4$); 5.25 (1H, m, $H^6$); 3.95 (1H, m, $H^{17'}$); 3.80 (6H, s, $OCH_3$ + $C^{23}OOCH_3$); 3.73 (1H, s, $H^2$); 3.6 (3H, s, $C^{18}OOCH_3$); 2.7 (3H, s, $NCH_3$); 1.28 (16H, broad peak, $CH_2$-); 0.88-0.8 (6H, $tCH_3 21$ + $tCH_3 21'$)

Spectre IR (KBr, $cm^{-1}$)

3430, 3040, 2930, 2860, 1770, 1735, 1710, 1615, 1505, 1460, 1410, 1370, 1230 -1250, 1000-1100.

Spectre de masse (DCI, isobutane) :

1089, 1064, 1048, 1034, 1000, 990, 976, 960.

Exemple 15 : 4-(N-maleoyl-12-amino dodecanoyl vinblastine.

A une solution de 699 mg (2.37 mM) d'acide N-maleoyl-12-amino dodecanoïque et de 433µl (3.95 mM) de N-methylmorpholine dans 4.2 ml d'acétate d'éthyle refroidie à 0°C, on ajoute goutte à goutte une solution de 307 µl (2.37 mM) d'isobutylchloroformiate dans 1 ml d'acétate d'éthyle.

On agite 3 minutes à 0°C puis on ajoute 607 mg (0.79 mM) de 0-4-deacetyl vinblastine en solution dans 1 ml d'acetate d'éthyle en gardant la température à 0°C. On laisse ensuite revenir à température ambiante et on agite le mélange pendant une nuit.

On filtre la solution et la phase acétate d'éthyle est évaporée à sec sous vide. Le résidu obtenu est purifié par chromatographie sur colonne de silice (élution éthanol / acetate d'éthyle 30/90). On obtient ainsi 271 mg de produit pur.

Rendement : 33%

Spectre RMN ($CDCl_3$, 360 MHz, ppm) :

8 (1H, s, NH ind$^{16'}$); 7.5-7.10 (4H, m, $H^{11'}H^{12'}H^{13'}H^{14'}$); 6.65 (2H, s, dl maléimide); 6.60 (1H, s, $H^{14}$); 6.08 - (1H, s, $H^{17}$); 5.83 (1H, m, $H^7$); 5.48 (1H, s, $H^4$); 5.25 (1H, d, $H^6$); 3.95 (1H, m, $H^{17'}$); 3.78 (6H, s, $-OCH_3$ + $C^{23}OOCH_3$); 3.73 (1H, s, $H_2$); 3.6 (3H, s, $C^{18'}OOCH_3$); 2.7 (3H, s, $NCH_3$); 1.25 (20H, m, $-CH_2-$); 0,9-0,8 (6H; t, $CH_3$21 + $CH_3$21').

Spectre IR (KBr, $cm^{-1}$) :

3460, 3040, 2930, 2860, 1735, 1700, 1610, 1500, 1460, 1430, 1410, 1365, 1245, 1220.

Spectre de masse (DCI, isobutane) :

1063, 1046 ($M^+$ +1), 1037, 1028, 1016, 312, 117.

Exemple 16 : 4-(N-maleoyl)-L-alanyl-lysyl éthyl ester -vinblastine

A une solution de 40.26 mg (0.163 mM) de lysine éthyl ester HCl et de 150 µl (0,978 mM) de triéthylamine dans 7,5 ml d'éthanol absolu, on ajoute goutte à goutte une solution de 150 mg (0,163 mM) de 4-(N-maléoyl)-L-alanyl-vinblastine dans 1 ml d'éthanol. On agite une nuit à température ambiante, puis on évapore la solution. Le résidu obtenu est purifié par chromatographie sur colonne de silice (élution 14% MeOH/$NH_3$ dans éther). On obtient ainsi 101,2 mg de produit pur.

Rendement : 67%

Spectre de masse (DCI, isobutane) :

1.095 ($M^+$ +2) ; 1.094 ($M^+$ +1) : 1.037 ; 921 ; 839 ; 769 ; 693 ; 615 ; 574 ; 532.

Spectre I.R. (KBr) $cm^{-1}$ : 3.460 ; 2.940 ; 1.740 ; 1.710 ; 1.615 ; 1.500 ; 1.460 ; 1.430 ; 1.590 ; 1.250 ; 1.225 ; 1.120 ; 1.010 ; 735.

Spectre RMN ($CDCl_3$, 360 MHz, ppm) : 8 (1H, NH, 16') ; 7.5-7.1 (4H, $H^{11'}$, $H^{12'}$, $H^{13'}$, $H^{14'}$) ; 6,6 (1H, s, $H^{14}$) ; 6,08 (1H, s, $H^{17}$) ; 5,8 (1H, m, $H^7$) ; 5,45 (1H, s, $H^4$) ; 5,3 (1H, m, $H^6$) ; 4,8 (1H, m, CH*) ; 4,18 (2H, q, -$OCH_2$) ; 3,83 (3H, s, $OCH_3$) ; 3,78 (3H, s, $OCH_3$) ; 3,6 (3H, s, OMe) ; 2,68 (3H, s, $NCH_3$) ; 1,65 (3H, d, $CH_3$ ala) ; 1,43 (2H, s) ; 1,28 ($CH_3$, $OCH_2CH_3$) ; 0,9-0,8 (6H, $CH_3^{-21}$ + $CH_3^{-21'}$).

Exemple 17 : Couplage de 4-(N-maléoyl)-L-alanyl vinblastine avec l'albumine humaine galactosylée (AHgal)- :($V_4$-Ala-Mal-AHg)

44 mg de 4-(N-maléoyl)-L-alanyl vinblastine sont dissous dans 1 ml de dioxanne. D'autre part, on prépare une solution de 50 mg d'AHgal dans 5 ml de tampon phosphate 0,2 M pH 8,5. La solution de 4-(N-maléoyl)-L-alanyl vinblastine est ajoutée à la solution d'AHgal. Le mélange est agité à température ordinaire pendant une nuit, purifié par filtration sur gel Sephadex G-25 (2,6 x 96 cm) équilibré dans une solution de NaCl 9‰ pH 7,5 . Le pic exclu est collecté (96 ml) et concentré par ultrafiltration et stérilisé.

Le contenu en protéines est mesuré par la méthode de Lowry et le contenu en alcaloïdes estimé par mesure de la radioactivité. Le conjugué obtenu contient 13,5 moles d'alcaloïde par mole d'albumine humaine galactosylée.

Exemple 18 : Couplage de 4-(N-maleoyl)-6-aminocaproyl vinblastine avec l'albumine humaine galactosylée (AHg) ($V_4$-$C_6$-Mal-AHg).

234 mg de 4-(N-maleoyl)-6-aminocaproyl vinblastine sont dissous dans 1 ml de dioxanne. D'autre part, on prépare une solution de 50 mg d'AHgal dans 5 ml de tampon phosphate 0,2 M pH 8,5. La solution de 4-(N-maleoyl)-6-aminocaproique vinblastine est ajoutée à la solution d'AHgal. Le mélange est agité à température ordinaire pendant une nuit et ensuite purifié par filtration sur gel Sephadex G-25 (2,6 x 96 cm) équilibré dans une solution de NaCl 9‰ pH 7,5. Le pic exclu est collecté (100 ml), concentré par ultrafiltration et stérilisé. Le contenu en protéines est mesuré par la méthode de Lowry et le contenu en alcaloïdes estimé par mesure de la radioactivité. Le conjugué obtenu contient 12.7 moles d'alcaloïde par mole d'albumine humaine galactosylée.

Exemple 19 : Couplage de 4-(N-maleoyl)-L-glutamyl $\gamma$ méthyl ester vinblastine avec l'albumine humaine galactosylée (AHg). ($V_4$-glu $\gamma$ méthyl ester-Mal-AHg)

24 mg de 4-(N-maleoyl)-L glutamyl $\gamma$ méthyl ester vinblastine sont dissous dans 1 ml de dioxanne. D'autre part, on prépare une solution de 50 mg d'AHgal dans 5 ml de tampon phosphate 0,2 M pH 8.5. La solution de 4-(N-maléoyl)-L glutamyl $\gamma$ méthyl ester vinblastine est ajoutée à la solution d'AHgal. Le mélange est agité à température ordinaire pendant une nuit et purifié par filtration sur gel Sephadex G-25 - (26 x 96 cm) équilibré dans une solution de NaCl 9‰ pH 7.5. Le pic exclu est collecté (100 ml) et concentré par ultrafiltration et stérilisé. Le contenu en protéines est mesuré par la méthode de Lowry et le contenu en alcaloïdes estimé par mesure de la radioactivité. Le conjugué obtenu contient 11,8 moles d'alcaloïde par mole d'albumine galactosylée.

Exemple 20 : Couplage de 4-(N-maleoyl)-L-Isoleucyl vinblastine avec l'albumine humaine galactosylée (AH gal). ($V_4$-Ile-Mal-AHg)

250 mg de 4-(N-maleoyl)-L-Isoleucyl vinblastine sont dissous dans 20 ml de dioxanne.

D'autre part, on prépare une solution de 591 mg d'AHgal dans 16.5 ml de tampon phosphate 0.4 M pH 8.5.

La solution de 4-(N-maleoyl)-L-Isoleucyl vinblastine est ajoutée à la solution d'AHgal. Le mélange est agité à température ordinaire pendant une nuit, purifié par filtration sur gel Sephadex G-25 (2.6 x 96 cm), équilibré dans une solution de NaCl 9 ‰ pH 7.5. Le pic exclu est collecté (180 ml), concentré par ultrafiltration et stérilisé.

Le contenu en protéine est mesuré par la méthode de Lowry et le contenu en alcaloïde est estimé par mesure de la radioactivité.

Le conjugué obtenu contient 8.8 moles d'alcaloïde par mole d'albumine humaine galactosylée.

Exemple 21 : Couplage de 4-(N-Maleoyl)-L-aspartyl $\alpha$ benzyl ester vinblastine avec l'albumine humaine galactosylée (AHgal). ($V_4$-Asp $\alpha$ B.E -Mal-AHg)

105 mg de 4-(N-Maleoyl)-L-aspartyl $\alpha$ benzyl ester vinblastine sont dissous dans 7.9 ml de dioxanne.

D'autre part, on prépare une solution de 226 mg d'AHgal dans 6.3 ml de tampon phosphate 0.4 M pH 8.5.

La solution de 4-(N-Maleoyl)-L-aspartyl benzyl ester vinblastine est ajoutée à la solution d'AHgal. Le mélange est agité à température ordinaire pendant une nuit, purifié par filtration sur gel Sephadex G-25 (2,6 x 96 cm), équilibré dans solution de NaCl 9 ‰ pH 7.5. Le pic exclu est collecté (60 ml), concentré par ultrafiltration et stérilisé.

Le contenu en protéine est mesuré par la méthode de Lowry et le contenu en alcaloïde estimé par mesure de la radioactivité.

Le conjugué obtenu contient 5.7 moles d'alcaloïde par mole d'albumine humaine galactosylée.

Exemple 22 : Couplage de 4-(N-maleoyl)-11 amino undecanoyl vinblastine avec l'albumine humaine galactosylée (AHgal). ($V_4$-$C_{11}$-Mal-AHg)

440 mg de 4-(N-maleoyl)-11 amino undecanoyl vinblastine sont dissous dans 46 ml de dioxanne.

D'autre part, on prépare une solution de 969 mg d'AHgal dans 27 ml de tampon phosphate 0.4 M pH 8.5.

Le solution de 4-(N-maleoyl)-11 amino undecanoyl vinblastine est ajoutée à la solution d'AHgal. Le mélange est agité à 40°C pendant 5 heures, purifié par filtration sur gel Trisacryl (5.6 x 50 cm) équilibré dans une solution de NaCl 9 ‰ pH 7.5. Le pic exclu est collecté (250 ml) et concentré par ultrafiltration et stérilisé.

Le contenu en protéine est mesuré par la méthode de Lowry et le contenu en alcaloïde estimé par mesure de la radioactivité.

Le conjugué obtenu contient 10.2 moles d'alcaloïde par mole d'albumine humaine galactosylée.

Exemple 23 : Couplage de 4-N-maleoyl-12-amino dodecanoyl vinblastine avec l'albumine humaine galactosylée (AHgal). ($V_4$-$C_{12}$-Mal-AHg)

120 mg de 4-(N-maleoyl)-12-amino dodecanoyl vinblastine sont dissous dans 16 ml de dioxanne.

D'autre part, on prépare une solution de 339 mg d'AHgal dans 9.4 ml de tampon phosphate 0.4 M pH 8.5.

La solution de 4-(N-maleoyl)-12-amino dodecanoyl vinblastine est ajoutée à la solution d'AHgal. Le mélange est agité à 40°C pendant 6 heures, purifié par filtration sur gel Trisacryl (5.6 x 50 cm) équilibré dans une solution de NaCl 9 ‰ pH 7.5. Le pic exclu (240 ml) est collecté, concentré par ultrafiltration et stérilisé.

Le contenu en protéine est mesuré par la méthode de Lowry et le contenu en alcaloïde estimé par mesure de la radioactivité. Le conjugué obtenu contient 9 moles d'alcaloïde par mole d'albumine humaine galactosylée.

Exemple 24 : Couplage de 4-(N-maleoyl)-L-alanyl vinblastine avec l'immunoglobuline non spécifique (IgG). - ($V_4$-Ala Mal-IgG)

4 mg de 4-(N-maleoyl)-L-alanyl vinblastine sont dissous dans 193 µl d'ethanol. D'autre part, on prépare une solution de 10 mg d'IgG (626 µl) dans 357 µl de tampon phosphate 0.4 M pH 8.5 et 447 µl d'eau (7 mg Prot/ml). La solution de 4-(N-maleoyl)-L-alanyl vinblastine est ajoutée à la solution d'IgG. Le mélange est agité à 35°C pendant une nuit et purifié par HPLC sur une colonne (gel filtration) Dupont de Nemours GF 450 + 250 équilibrée dans un tampon phosphate 0.2 M pH 7.5.

Le pic exclu est collecté et dosé.

Le contenu en protéine est mesuré par la méthode de Lowry et le contenu en alcaloïde est estimé par mesure de la radioactivité. Le conjugué obtenu contient 6.6 moles d'alcaloïde par mole d'immunoglobuline.

Les composés de l'invention ont été soumis à une étude pharmacologique.

1. Sensibilité aux enzymes lysosomiales.

La sensibilité des conjugués aux enzymes lysosomiales a été étudiée par incubation, pendant 48 heures à 37°C, de ces conjugués en présence de cystéine 5 mM, de tampon acétate 40 mM et d'enzymes lysosomiales.

Après 48 heures d'incubation, les protéines non dégradées sont précipitées, après addition de 5 mg d'albumine sérique, par 2 volumes d'acétonitrile.

Après incubation des échantillons à 4°C pendant 40′ et centrifugation à 3000 RPM pendant 40′, la radioactivité du surnageant est estimée par comptage d'une partie aliquote en scintillation liquide. La radioactivité soluble est une mesure de la digestion du conjugué.

Les pourcentages de digestion obtenus sont les suivants :
$V_4$-Ala-Mal AHg : 92%
$V_4$-$C_6$-Mal AHg : 100%
$V_4$-Ile-Mal AHg : 75%
$V_4$-$C_{11}$-Mal AHg : 79%
$V_4$-$C_{12}$-Mal AHg : 92%

2. Stabilité dans le sérum.

La stabilité des conjugués en présence de sérum a été étudiée par incubation du conjugué en présence de 62% de sérum de veau foetal, à 37°C pendant 48 heures.

Après 48 heures d'incubation, les protéines non dégradées sont précipitées par addition d'un volume d'acide trichloroacétique (TCA 40%).

Après incubation des échantillons à 4°C pendant une heure, ceux-ci sont centrifugés et la radioactivité du surnageant est estimée par comptage d'une partie aliquote en scintillation liquide.

La radioactivité soluble est une mesure de la digestion du conjugué.

Les résultats montrent que les conjugués sont stables au cours des 48 heures.

3. Stabilité à pH acide.

La stabilité des conjugués à pH acide a été étudiée par incubation du conjugué en présence de tampon acétate 40 mM, pH 4.5, à 37°C, pendant 48 heures. La digestion est estimée par la technique de précipitation au TCA.

Les résultats montrent que les conjugués sont stables dans ces conditions.

4. Activité chimiothérapeutique sur leucémie P 388.

L'activité chimiothérapeutique des dérivés intermédiaires et des conjugués a été évaluée sur la leucémie P 388 administrée par voie i.p. chez la souris $BDF_1$ femelle : $10^6$ cellules tumorales sont inoculées par voie i.p. au jour 0. Le conjugué est administré par voie i.p. au jour 1.

La valeur de l'ILS représente le pourcentage du temps de survie des souris traitées comparativement à des souris non traitées.

Le rapport molaire indique le nombre de moles d'alcaloïde par mole d'albumine humaine galactosylée.

Le nombre de souris survivantes au jour 60 est indiqueé ainsi que la dose en mg/kg de l'alcaloïde et de la protéine.

3.1. Dérivés intermédiaires.

| Produit | Dose mg/kg/j | ILS % | Survivants au jour 60 |
|---|---|---|---|
| VBL (vinblastine) | 3 | 77 | 0/10 |
| VCR (vincristine) | 2,7 | 64 | 0/11 |
| $V_4$-Ala-Mal 5(exemple 9) | 25 | 100 | 0/7 |
| | 50 | 127 | 0/7 |
| | 75 | - 70 | 0/6 |
| $V_4$-Glu- γ-methyl ester-Mal (ex 11) | 50 | 88 | 0/10 |
| | 100 | 111 | 0/10 |
| $V_4$-Ile-Mal (exemple 12) | 50 | 148 | 0/7 |
| | 100 | - 79 | 0/7 |
| $V_4$-$C_6$-Mal (exemple 10) | 25 | 102 | 0/8 |
| | 50 | 218 | 1/7 |
| | 100 | - 83 | 0/7 |
| $V_4$-$C_{11}$-Mal (exemple 14) | 50 | 37 | 1/7 |
| | 100 | - 19 | 1/5 |
| $V_4$-$C_{12}$-Mal (exemple 15) | 25 | 185 | 1/5 |
| | 50 | > 633 | 6/8 |
| | 100 | 7 | 1/7 |

3.2. Conjugués.

| Produit | Dose mg/kg/j Vinca | Protéine | Rapport molaire | ILS % | Survivants jour 60 |
|---|---|---|---|---|---|
| VBL | 3 | | | 77 | 0/10 |
| VCR | 2,7 | | | 64 | 0/11 |
| $V_4$-Ala-Mal AHg | 60 | 374-466 | 13.5-10.7 | 69 | 0/5 |
| | 80 | 450 | 15 | 133 | 1/5 |
| | 100 | 562 | 15 | 201 | 0/5 |
| | 130 | 2766 | 6.6 | 175 | 1/3 |
| | 150 | 3035 | 4.1 | 138 | 0/7 |
| | 150 | 1276 | 9.8 | > 552 | 3/5 |
| | 150 | 913 | 13.7 | > 574 | 3/5 |
| $V_4$-Asp-α-benzyl ester-Mal-AHg | | | | | |
| | 60 | 768 | 5.7 | 47 | 0/5 |
| $V_4$-Glu-γ-methyl ester-Mal AHg | | | | | |
| | 60 | 987 | 4.7 | 67 | 0/5 |
| | 100 | 1645 | 4.7 | 75 | 0/5 |
| | 150 | 2468 | 4.7 | 88 | 0/5 |
| $V_4$-Ile-Mal AHg | 60 | 545 | 8.8 | 51 | 0/5 |
| | 100 | 908 | 8.8 | 48 | 0/5 |
| | 150 | 1362 | 8.8 | 79 | 0/5 |

../...

| | | | | | |
|---|---|---|---|---|---|
| $V_4$-$C_6$-Mal AHg | 60 | 400 | 12 | 95 | 0/5 |
| | 150 | 995 | 12 | 137 | 1/5 |
| $V_4$-$C_{11}$-Mal AHg | 60 | 378 | 11,8 | 147 | 1/5 |
| | 80 | 504 | 11,8 | 153 | 1/5 |
| | | 753 | 7,9 | | |
| | 90 | 658 | 10,2 | 227 | |
| | 100 | 630 | 11,8 | > 606 | 3/5 |
| | 100 | 646 | 7,9 | 186 | 3/10 |
| | 150 | 945 | 11,8 | - 45 | 0/5 |
| $V_4$-$C_{12}$-Mal AHg | 90 | 741 | 9 | > 769 | 5/5 |

## Revendications

1. Conjugués d'alcaloïde vinca de type indoledihydroindole avec une protéine ou un fragment de protéine, répondant à la formule générale

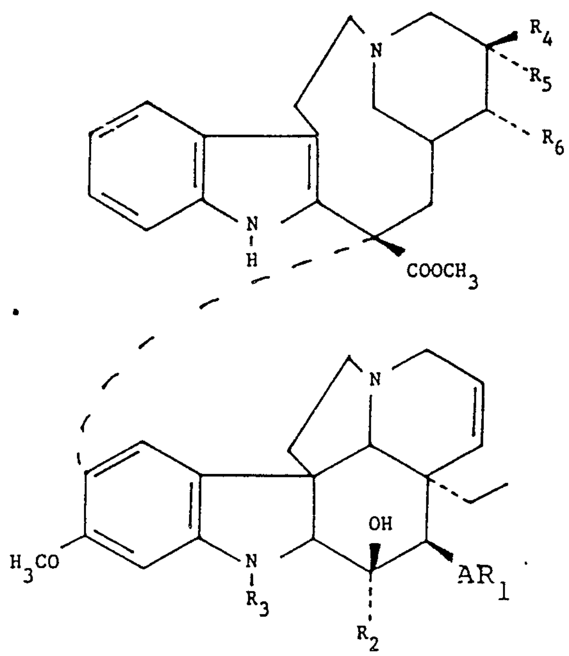

dans laquelle $R_1$ représente une protéine ou un fragment de protéine;
$R_2$ est $COOC_{1-3}$ alkyle, ou CO-$R_7$ où $R_7$ est $NH_2$, un ester d'acide aminé ou de peptide; $R_3$ est H, $CH_3$, CHO;
lorsque $R_5$ et $R_6$ sont pris séparément, $R_6$ est H et un des $R_4$ et $R_5$ est éthyl et l'autre est H ou OH;
lorsque $R_5$ et $R_6$ sont pris ensemble avec les carbones auxquels ils sont attachés, ils forment un noyau oxirane et $R_4$ est éthyle et
A est un dérivé organique bifonctionnel du type maléoylaminoacide ou maléoylpeptide ou maléoylphénoxy.

2. Conjugué selon la revendication 1 caractérisé en ce que A répond à la formule générale

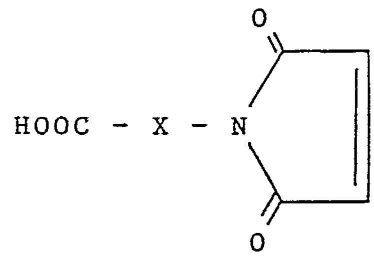

dans laquelle X représente une chaîne linéaire alkylène de 1 à 12 atomes de carbone,
une chaîne ramifiée alkylène de 2 à 5 atomes de carbone,
une chaîne cycloalkylène de 3 à 6 atomes de carbone,
une chaîne phénylène de 3 à 6 atomes de carbone,
ou le groupement R-CH des acides aminés naturels

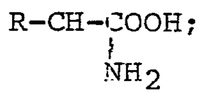

un fragment de chaîne peptidique du type $-(X_1-NH-CO)_n-X_1-$dans laquelle n = 1, 2, 3, 4 et $X_1$ est une chaîne linéaire alkylène de 1 à 12 atomes de carbone, une chaîne ramifiée alkylène de 2 à 5 atomes de carbone, une chaîne cycloalkylène de 3 à 6 atomes de carbone, une chaîne phénylène de 3 à 6 atomes de carbone, ou le groupement R-CH des acides aminés naturels ou encore un radiacalphényle,
sous sa forme racémique ou sous une de ses formes optiquement actives.

3. Conjugué selon la revendication 1 caractérisé en ce que le ou les groupements fonctionnalisés RCH des acides aminés naturels, représentés par X ou $X_1$, sont protégés par des groupements protecteurs connus en soi.

4. Conjugué selon l'une quelconque des revendications précédentes caractérisé en ce que le protéine $R_1$ est l'albumine de sérum bovin ou humain, la fétuine ou des immoglobulines.

5. Conjugué selon l'une quelconque des revendications précédentes caractérisé en ce que la protéine $R_1$ est traitée sélectivement.

6. Conjugué selon l'une quelconque des revendications précédentes caractérisé en ce que $R_1$ est une immunoglobuline choisie parmi le groupe constitué par

-Ig de chèvre ou de mouton immunisés avec l'antigène carcinoembryonnaire;
-Ig de lapin anti-LLA;
-différentes Ig de singe anti-LLA, anti-LMA, anti-LLC, anti-LMC;
-Ig de chèvre ou de mouton immunisés avec des membranes de carcinome du poumon;
-Ig monoclonales à partir d'hybridomes de souris secrétant des anticorps contre le carcinome colo-rectal humain;
- Ig monoclonales à partir d'hybridomes de souris secrétant des anticorps contre le mélanome humain;
-Ig monoclonales à partir d'hybridomes de souris secrétant des anticorps réagissant avec des cellules leucémiques humaines;
-Ig monoclonales à partir d'hybridomes de souris secrétant des anticorps réagissant avec des cellules de neuroblastomes humaines;
-Ig monoclonales à partir d'hybridomes de souris secrétant des anticorps réagissant avec des antigènes de cancer du sein humain;
-Ig monoclonales à partir d'hybridomes de souris secrétant des anticorps réagissant avec des cellules de carcinome ovarien humain;
-Ig monoclonales à partir d'hybridomes de souris secrétant des anticorps réagissant avec des cellules d'ostéosarcome humain;
-Ig monoclonales à partir d'hybridomes de souris secrétant des anticorps du cancer du poumon;

ou un fragment d'immunoglobuline à savoir Fab, Fab' ou $F(ab')_2$ ou 1gM monomère obtenu à partir d'un anticorps par digestion par un enzyme protéolytique.

7. Procédé pour la préparation d'un conjugué d'alcaloïde vinca avec une protéine ou un fragment de protéine répondant à la formule (I)

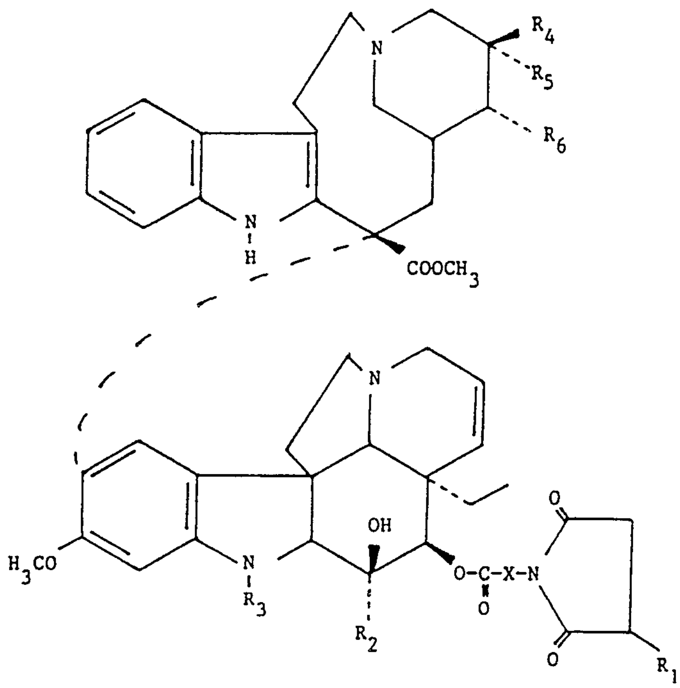

I

dans laquelle $R_1$, $R_2$, $R_3$, $R_4$, $R_5$, $R_6$ et X ont les significations mentionnées aux revendications 1 et 2, caractérisé en ce qu'on condense un maléoylaminoacide ou maléoylpeptide de formule

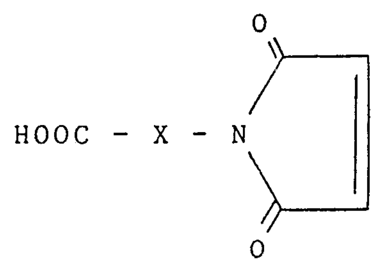

sur l'hydroxyle en $C_4$ d'un composé de formule générale (II)

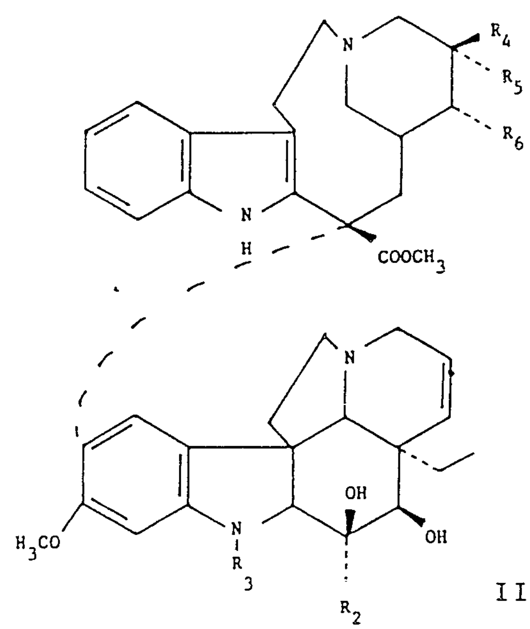

II

dans laquelle $R_2$, $R_3$, $R_4$, $R_5$, $R_6$ et X ont les significations mentionnées, et en ce qu'on condense le produit

intermédiaire obtenu avec une protéine ou un fragment de protéine.

8. Procédé selon la revendication 7 caractérisé en ce qu'on effectue la première étape de condensation au moyen d'un chloroformiate d'alkyle, de préférence le chloroformiate d'éthyle ou d'isobutyle, en présence d'une base aminée, telle que la triéthylamine, la N-méthyl-pipéridine ou la N-méthylmorpholine, dans un solvant organique tel que l'acétate d'éthyle, le tétrahydrofuranne ou le chlorure de méthylène, et en ce qu'on isole le composé intermédiaire obtenu du milieu réactionnel, en ce qu'on purifie et en ce qu'on effectue la deuxième étape de condensation de manière classique, en milieu aqueux, à une température comprise entre 4°C et 25°C et un pH de 7,5 à 9,5.

9. A titre de composé intermédiaire, le composé de formule générale (III)

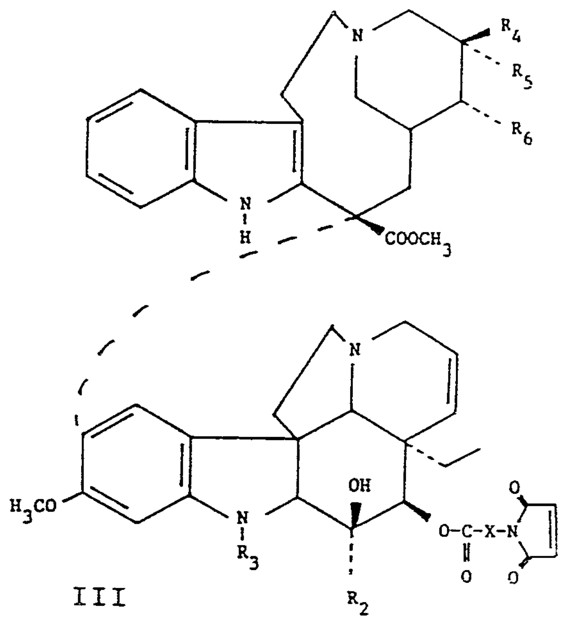

III

dans laquelle $R_2$, $R_3$, $R_4$, $R_5$, $R_6$ et X ont les significations mentionnées aux revendications 1 et 2.

10. Composition pharmaceutique comportant, à titre de substance active, le conjugué selon l'une quelconque des revendications 1 à 6 en combinaison avec des supports et excipients pharmaceutiquement acceptables selon une dose unitaire variant de 50 mg à plusieurs grammes, éventuellement en combinaison avec d'autres substances actives.

11. Composition pharmaceutique selon la revendication 10 comportant le conjugué selon l'une quelconque des revendications 1 à 6 à l'état dissous dans un solvant pharmaceutiquement acceptable, tel que l'eau physiologique ou des solutions tamponnées au moyen d'un tampon phosphate.

12. Utilisation du conjugué selon l'une quelconque des revendications 1 à 6 pour la préparation de médicaments à activité anti-tumorale et cytostatique.

Revendications pour les Etats contractants suivants: AT,ES,GR

1. Procédé pour la préparation d'un conjugué d'alcaloïde vinca avec une protéine ou un fragment de protéine répondant à la formule (I)

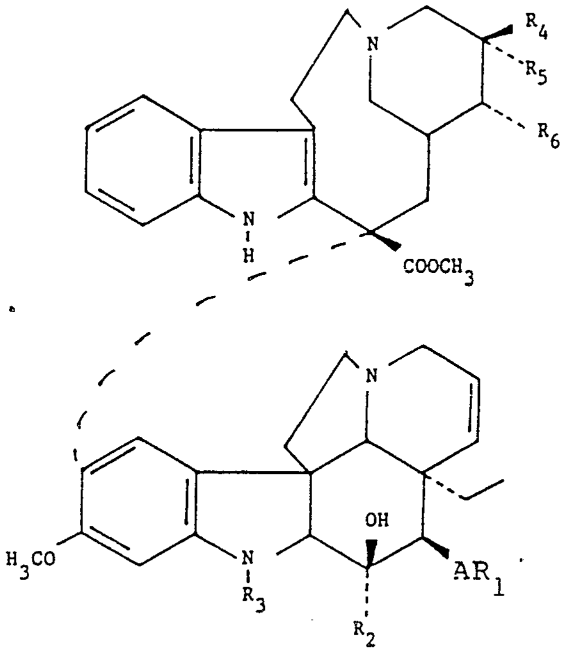

dans laquelle $R_1$ représente une protéine ou un fragment de protéine;
$R_2$ est $COOC_{1-3}$ alkyle, ou CO-$R_7$ où $R_7$ est $NH_2$, un ester d'acide aminé ou de peptide; $R_3$ est H, $CH_3$, CHO;
lorsque $R_5$ et $R_6$ sont pris séparément, $R_6$ est H et un des $R_4$ et $R_5$ est éthyl et l'autre est H ou OH;
lorsque $R_5$ et $R_6$ sont pris ensemble avec les carbones auxquels ils sont attachés, ils forment un noyau oxirane et $R_4$ est éthyle et
A est un dérivé organique bifonctionnel du type maléoylaminoacide ou maléoylpeptide ou maléoylphénoxy caractérisé en ce qu'on condense un maléoylaminoacide ou maléoylpeptide ou maléoylphénoxy de formule

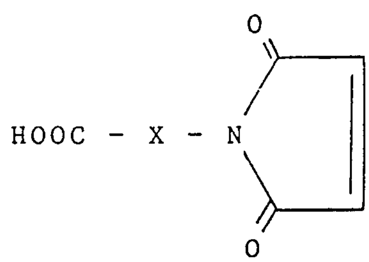

dans laquelle X représente une chaîne linéaire alkylène de 1 à 12 atomes de carbone,
une chaîne ramifiée alkylène de 2 à 5 atomes de carbone,
une chaîne cycloalkylène de 3 à 6 atomes de carbone,
une chaîne phénylène de 3 à 6 atomes de carbone, ou le groupement R-CH des acides aminés naturels

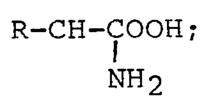

un fragment de chaîne peptidique du type -($X_1$-NH-CO)$_n$-$X_1$-dans laquelle n = 1, 2, 3, 4 et $X_1$ est une chaîne linéaire alkylène de 1 à 12 atomes de carbone, une chaîne ramifiée alkylène de 2 à 5 atomes de carbone, une chaîne cycloalkylène de 3 à 6 atomes de carbone, une chaîne phénylène de 3 à 6 atomes de carbone, ou le groupement R-CH des acides aminés naturels ou encore un radicalphényle
sous sa forme racémique ou sous une de ses formes optiquement actives
sur l'hydroxyle en $C_4$ d'un composé de formule générale (II)

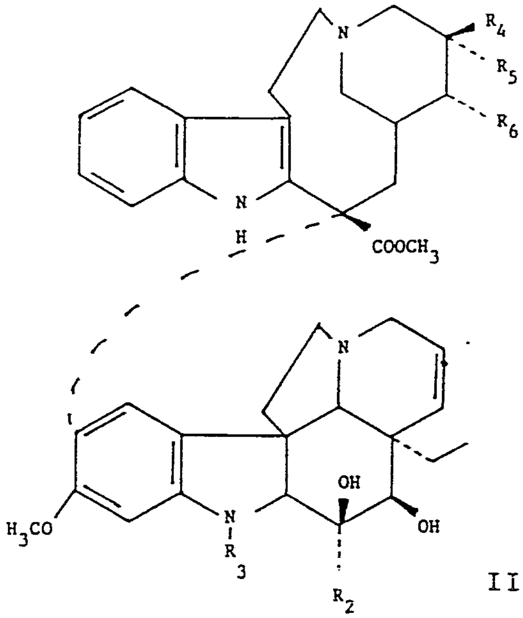

II

dans laquelle $R_2$, $R_3$, $R_4$, $R_5$, $R_6$ et X ont les significations mentionnées, et en ce qu'on condense le produit intermédiaire obtenu avec une protéine ou un fragment de protéine.

2. Procédé selon la revendication 1 caractérisé en ce qu'on effectue la première étape de condensation au moyen d'un chloroformiate d'alkyle, de préférence le chloroformiate d'éthyle ou d'isobutyle, en présence d'une base aminée, telle que la triéthylamine, la N-méthyl-pipéridine ou la N-méthylmorpholine, dans un solvant organique tel que l'acétate d'éthyle, le tétrahydrofuranne ou le chlorure de méthylène, et en ce qu'on isole le composé intermédiaire obtenu du milieu réactionnel, en ce qu'on purifie et en ce qu'on effectue la deuxième étape de condensation de manière classique, en milieu aqueux, à une température comprise entre 4°C et 25°C et un pH de 7,5 à 9,5.

3. Procédé selon la revendication 1 ou 2 caractérisé en ce que le ou les groupements fonctionnalisés RCH des acides aminés naturels, représentés par X ou $X_1$, sont protégés par des groupements protecteurs connus en soi.

4. Procédé selon l'une quelconque des revendications précédentes caractérisé en ce que la protéine $R_1$ est l'albumine de sérum bovin ou humain, la fétuine ou des immoglobulines.

5. Procédé selon l'une quelconque des revendications précédentes caractérisé en ce que la protéine $R_1$ est traitée sélectivement.

6. Procédé selon l'une quelconque des revendications précédentes caractérisé en ce que $R_1$ est une immunoglobuline choisie parmi le groupe constitué par

-Ig de chèvre ou de mouton immunisés avec l'antigène carcinoembryonnaire;

-Ig de lapin anti-LLA;

-Ig de chèvre ou de mouton immunisés avec l'antigène carcinoembryonnaire;

-Ig de lapin anti-LLA;

-différentes Ig de singe anti-LLA, anti-LMA, anti-LLC, anti-LMC;

-Ig de chèvre ou de mouton immunisés avec des membranes de carcinome du poumon;

-Ig monoclonales à partir d'hybridomes de souris secrétant des anticorps contre le carcinome colo-rectal humain;

-Ig monoclonales à partir d'hybridomes de souris secrétant des anticorps contre le mélanome humain;

-Ig monoclonales à partir d'hybridomes de souris secrétant des anticorps réagissant avec des cellules leucémiques humaines;

-Ig monoclonales à partir d'hybridomes de souris secrétant des anticorps réagissant avec des cellules de neuroblastomes humaines;

-Ig monoclonales à partir d'hybridomes de souris secré tant des anticorps réagissant avec des antigènes de cancer du sein humain;

-Ig monoclonales à partir d'hybridomes de souris secrétant des anticorps réagissant avec des cellules de carcinome ovarien humain;

-Ig monoclonales à partir d'hybridomes de souris secrétant des anticorps réagissant avec des cellules d'ostéosarcome humain;

-Ig monoclonales à partir d'hybridomes de souris secrétant des anticorps du cancer du poumon;
ou un fragment d'immunoglobuline à savoir Fab, Fab' ou $F(ab')_2$ ou1gM monomère obtenu à partir d'un anticorps par digestion par un enzyme protéolytique.

7. procédé de prépraration du composé intermédiaire de formule générale (III)

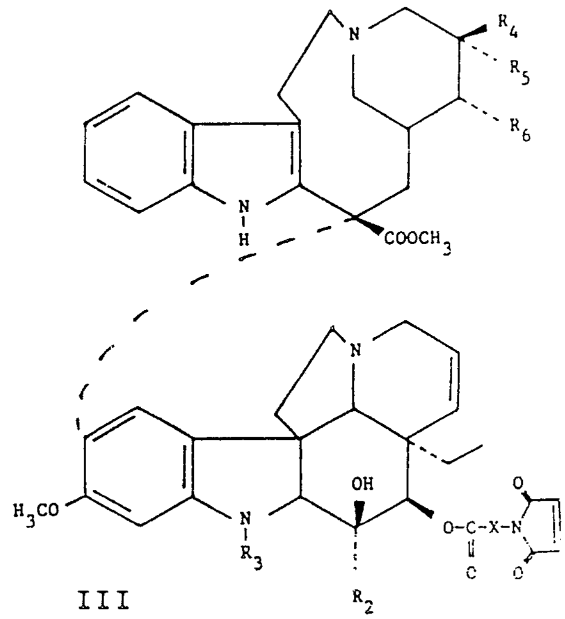

III

dans laquelle $R_2$, $R_3$, $R_4$, $R_5$, $R_6$ et X ont les significations mentionnées aux revendications 1 et 2, caractérisé en ce qu'on condense un maléoylaminoacide, ou maléoylaminopeptide ou maléoylphénoxy de formule

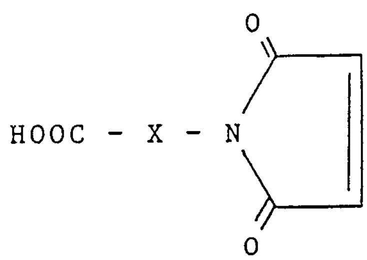

dans laquelle X a les significations mentionnées dans le revendication 1 sur l'hydroxyle en $C_4$ du composé de formule (II).

8. Procédé pour la préparation d'une composition pharmaceutique caractérisé en ce qu'on mélange le composé de formule (I) avec des supports et excipients pharmaceutiquement acceptables selon une dose unitaire variant de 50 mg à plusieurs grammes, éventuellement en combinaison avec d'autres substances actives.